# EUROPÄISCHE PATENTANMELDUNG

(11) **EP 3 620 100 A1**
(43) Veröffentlichungstag der Anmeldung: **11.03.2020**
(21) Anmeldenummer: 18193150.2
(22) Anmeldetag: 07.09.2018
(51) Int. Cl.: A61B 1/247, A61B 1/06, A61B 1/00

(54) **ZAHNÄRZTLICHES KAMERAHANDSTÜCK ZUM ERSTELLEN INTRAORALER AUFNAHMEN**

(71) Anmelder: Kaltenbach & Voigt GmbH, 88400 Biberach (DE)
(72) Erfinder: Maurer, Chris, 89143 Blaubeuren (DE)
(74) Vertreter: Thun, Clemens

(57) **Zusammenfassung**

Ein zahnärztliches Kamerahandstück (100) zum Erstellen intraoraler Aufnahmen weist Beleuchtungsmittel (10) zum Beleuchten eines vor einem Eintrittsfenster (9) des Kamerahandstücks (100) befindlichen Objekts (200), ein optisches System (15) zum Abbilden des Objekts (200) auf eine Bildsensor-Anordnung (20), sowie eine Bildsensor-Anordnung (20) auf, welche zumindest zwei getrennte digitale Bildsensoren (21, 22, 23) aufweist. Ferner sind Bildteilungsmittel (16) vorhanden, welche das Objekt (200) auf jeden der Bildsensoren (21, 22, 23) abbilden, sowie Filtermittel (18), welche derart ausgebildet sind, dass das Objekt (200) auf jedem Sensor (21, 22, 23) jeweils in einem vorgegebenen Wellenlängenbereich (I, II, III) abgebildet wird.

## Beschreibung

Die vorliegende Erfindung betrifft ein zahnärztliches Kamerahandstück, welches zum Erstellen intraoraler Aufnahmen vorgesehen ist.

Digitale zahnärztliche Kameras, mit deren Hilfe Aufnahmen von Zähnen oder anderen Objekten im Mundraum eines Patienten erstellt werden können, finden in der Zahnmedizin zunehmend Verwendung. Ein großer Vorteil derartiger Kameras besteht darin, dass die hiermit erstellten Aufnahmen unmittelbar beispielsweise auf einem Display eines zahnärztlichen Behandlungsplatzes dargestellt werden können und dementsprechend gegebenenfalls erforderliche Behandlungsmaßnahmen einem Patienten anschaulich erläutert werden können. Auch das Dokumentieren von Untersuchungs- und Behandlungsergebnissen wird mit Hilfe einer derartigen Intraoralkamera deutlich vereinfacht. Dabei können mit Hilfe moderner zahnärztlicher Kameras sowohl Einzelaufnahmen beziehungsweise Standbilder als auch Videos erstellt werden.

In klassischer Weise werden mit Hilfe der derzeit bekannten Intraoralkameras sogenannte Weißlichtaufnahmen erstellt. Das heißt, der von der Optik der Kamera erfasste Bereich wird mit einer Weißlichtquelle beleuchtet und das von der Kamera erfasste Farbbild setzt sich aus den Wellenlängen des sichtbaren Bereichs zusammen. Damit entspricht das von der Kamera erstellte Bild im Wesentlichen der Sichtweise eines menschlichen Beobachters, wobei das Platzieren der Kamera in unmittelbarer Nähe des zu betrachtenden Objekts, also beispielsweise eines Zahns, das Erkennen einzelner Details ermöglicht.

Neben dem Erstellen derartiger Weißlichtaufnahmen existieren in der Zahnmedizin allerdings auch weitere optische Diagnoseverfahren, mit deren Hilfe beispielsweise das frühzeitige Erkennen von Karies unterstützt werden soll.

Ein erstes bekanntes Diagnoseverfahren ist hierbei die sogenannte Transillumination, bei der ein zu untersuchender Zahn durchleuchtet wird und wiederum mit Hilfe einer Kamera erfasst wird, in welcher Weise der Zahn das in ihn eingekoppelte Licht streut. Dabei wird die Tatsache genutzt, das kariöses Zahngewebe Licht in anderer Weise streut als gesundes Zahngewebe. Diese Vorgehensweise ist somit vergleichbar zu einer klassischen Röntgenaufnahme eines Zahns, wobei allerdings im Vergleich zur Röntgenaufnahme bei der Transillumination Licht einer deutlich längeren Wellenlänge verwendet wird und dementsprechend die zum Durchleuchten des Zahns verwendete Strahlung weniger problematisch im Hinblick auf eventuelle gesundheitliche Nebenwirkungen ist.

Ein entsprechendes zahnärztliches System zum Transilluminieren von Zähnen ist beispielsweise aus der EP 2 452 614 A1 der Anmelderin bekannt. Es hat sich hierbei herausgestellt, dass die Qualität der im Rahmen eines derartigen Transilluminationsverfahrens erhaltenen Aufnahmen dann besonders gut ist, wenn der Zahn nicht mit sichtbaren Licht, sondern stattdessen mit Licht im nahen Infrarotbereich durchleuchtet wird und auch diese gestreute, hinsichtlich ihrer Wellenlänge im Wesentlichen nicht veränderte Infrarotstrahlung erfasst wird.

Ein weiteres, zum Erkennen von Karies genutztes optisches Verfahren beruht auf einer sogenannten Fluoreszenzdiagnose. Diese Fluoreszenzdiagnose beruht auf der Erkenntnis, dass kariöses Zahngewebe beim Beleuchten mit einer Anregungsstrahlung geeigneter Wellenlänge fluoresziert und Licht in Form von Fluoreszenzstrahlung abgibt. Wird also ein Zahn mit einer entsprechenden Anregungsstrahlung großflächig beleuchtet, so werden diejenigen Bereiche verstärkt Fluoreszenzstrahlung abgeben, in denen Karies vorhanden ist.

Entsprechende Fluoreszenzdiagnose-Verfahren sind im Stand der Technik vielfach und seit langer Zeit bekannt, wobei sich herausgestellt hat, dass dieses Verfahren insbesondere bei der Verwendung einer Anregungsstrahlung aus dem ultravioletten Bereich zu guten Ergebnissen führt. Die als Antwort hierauf entstehende Fluoreszenzstrahlung weist dann allerdings eine geringere Energie, also eine etwas längere Wellenlänge auf, und liegt hierbei dann üblicherweise im Bereich des sichtbaren Lichts.

Während im Rahmen des oben erläuterten Transilluminationsverfahrens primär tieferliegende Karies aufweisende Bereiche erkannt werden können, kann mit Hilfe des Fluoreszenzdiagnose-Verfahrens in erster Linie an der Zahnoberfläche beziehungsweise Außenseite eines Zahns vorhandenes kariöses Gewebe erkannt werden. Beide Vorgehensweisen ergänzen sich also zu einem gewissen Grad, so dass eine Kombination beider Verfahren zur Optimierung einer berührungslosen Kariesdiagnose von Vorteil wäre.

Im Stand der Technik wird bereits vorgeschlagen, zwei der oben beschriebenen Diagnose-Verfahren innerhalb eines Geräts, also in einer Kamera zu realisieren. Die Erstellung einer Weißlichtaufnahme wird hier ebenfalls als Diagnose-Verfahren angesehen, sodass also grundsätzlich drei Diagnose-Verfahren beziehungsweise Aufnahmemodi denkbar sind, das Erstellen einer Weißlichtaufnahme, das Erstellen einer Transilluminationsaufnahme sowie das Erstellen einer Aufnahme im Rahmen einer Fluoreszenzdiagnose.

In diesem Zusammenhang wird beispielsweise in der DE 10 2006 041 020 A1 vorgeschlagen, die Transillumination eines Zahns mit einer Fluoreszenzdiagnose zu kombinieren. Die DE 100 43 749 A1 wiederum beschreibt das parallele Erfassen eines optischen Bilds im Rahmen einer Weißlichtaufnahme sowie zusätzlich auch eines Fluoreszenzbilds. In der DE 10 2013 006 636 A1 wiederum wird die Kombination des Erstellens eines optischen Bildes mit einem Bild basierend auf einem Transilluminationsverfahren oder einem Fluoreszenzdiagnoseverfahren vorgeschlagen.

Grundsätzlich wäre die Kombination aller drei Diagnose-Verfahren im Rahmen einer einzigen intraoralen Kamera wünschenswert, da - wie erläutert - alle Verfahren jeweils unterschiedliche Informationen hinsichtlich eines zu untersuchenden Zahns liefern. Ferner wäre es wünschenswert, wenn Aufnahmen in verschiedenen Aufnahmemodi zeitgleich erstellt werden können, da dann ein unmittelbares Kombinieren der Untersuchungsergebnisse möglich wäre.

Es hat sich allerdings gezeigt, dass das gleichzeitige Realisieren aller drei Aufnahmemodi nicht ohne Weiteres durchführbar ist. Grund hierfür ist zunächst, dass bei jeder Aufnahmeart der zu untersuchende Zahn mit Licht einer anderen spektralen Zusammensetzung zu beleuchten ist. Entscheidendes Problem ist allerdings, dass abhängig vom jeweiligen Aufnahmemodus vorzugsweise Licht nur eines bestimmten Wellenlängenbereichs erfasst werden sollte. Die hierbei jeweils bevorzugten Bereiche grenzen teilweise nahe aneinander an, sodass beispielsweise das Erfassen eines Bildes für eine Weißlichtaufnahme sowie eines Bildes für eine Fluoreszenzdiagnose mit Hilfe eines einzigen optischen Systems sowie eines zugehörigen einzelnen Bildsensors nicht ohne Weiteres möglich ist.

Aus dem Stand der Technik ist in diesem Zusammenhang bekannt, je nach Aufnahmeart entsprechende Filter einzusetzen, die dem Bildsensor vorgeschaltet sind. Dies erfordert dann allerdings, dass im optischen System der Kamera entsprechend verstellbare beziehungsweise auswechselbare Filterkomponenten vorhanden sein müssen. Dies führt nicht nur zu einem komplexeren mechanischen Aufbau der Kamera, sondern kann auch zu einer erhöhten Fehleranfälligkeit des optischen Systems führen. Weiterhin ist dann jeweils bei einem Wechsel zwischen zwei verschiedenen Aufnahmemodi ein entsprechender Wechsel des Filters erforderlich, was grundsätzlich gegen eine parallele Bildaufnahme in verschiedenen Aufnahmemodi spricht.

Der vorliegenden Erfindung liegt deshalb die Aufgabenstellung zu Grunde, ein zahnärztliches Kamerahandstück zum Erstellen intraoraler Aufnahmen zur Verfügung zu stellen, welches das parallele, also zeitgleiche Erstellen von Aufnahmen in zumindest zwei verschiedenen Aufnahmemodi ermöglicht.

Die Aufgabe wird durch ein zahnärztliches Kamerahandstück mit den Merkmalen des Anspruchs 1 gelöst. Vorteilhafte Weiterbildungen der Erfindung sind Gegenstand der abhängigen Ansprüche.

Die erfindungsgemäße Lösung beruht auf dem Gedanken, anstelle eines einzelnen Bildsensors eine Bildsensor-Anordnung zu verwenden, die zumindest zwei getrennte digitale Bildsensoren umfasst. Das optische System weist geeignete sog. Bildteilungsmittel auf, die das Objekt jeweils auf jeden der Bildsensoren abbilden, wobei ferner Filtermittel vorhanden sind, die derart ausgebildet sind, dass das Objekt auf jeden der Sensoren jeweils in einem vorgegebenen Wellenlängenbereich abgebildet wird, wobei sich die Wellenlängenbereiche für die Bildsensoren unterscheiden.

Erfindungsgemäß wird also ein zahnärztliches Kamerahandstück zum Erstellen intraoraler Aufnahmen vorgeschlagen, welches aufweist:
- Beleuchtungsmittel zum Beleuchten eines vor einem Eintrittsfenster des Kamerahandstücks befindlichen Objekts,
- ein optisches System zum Abbilden des Objekts auf eine Bildsensor-Anordnung,
- eine Bildsensor-Anordnung, welche zumindest zwei getrennte digitale Bildsensoren zum Erfassen des mit Hilfe des optischen Systems abgebildeten Objekts aufweist,
wobei das optische System Bildteilungsmittel aufweist, welche das Objekt auf jeden der Bildsensoren abbilden, sowie Filtermittel, welche derart ausgebildet sind, dass das Objekt auf jeden Sensor jeweils in einem vorgegebenen Wellenlängenbereich abgebildet wird, wobei sich die Wellenlängenbereiche für die Bildsensoren unterscheiden.

Durch die erfindungsgemäße Lösung wird das Problem gelöst, dass ein einzelner Sensor nicht gleichzeitig Wellenlängenbereiche, die zum Erstellen von Aufnahmen in unterschiedlichen Aufnahmemodi benötigt werden, erfassen kann. Dadurch, dass das Bild des zu untersuchenden Objekts - z.B. eines Zahns - mit Hilfe der Bildteilungsmittel aufgeteilt und durch mehrere Sensoren jeweils in verschiedenen Wellenlängenbereichen erfasst wird, besteht die Möglichkeit, zeitgleich unterschiedliche und unabhängige Wellenlängeninformationen hinsichtlich des von dem Objekt abgegebenen Lichts zu erfassen, sodass auch tatsächlich erstmalig nunmehr zeitgleich Aufnahmen in verschiedenen Aufnahmemodi erstellt werden können. Dies eröffnet insbesondere auch die Möglichkeit, parallel Videoaufnahmen in den unterschiedlichen Aufnahmemodi zu erstellen und beispielsweise nebeneinander oder überlagert auf einem Bildschirm eines zahnärztlichen Behandlungsplatzes darzustellen.

Dabei muss das von einem einzelnen Sensor der Bildsensor-Anordnung erfasste Signal nicht zwingend einer Aufnahme in einem bestimmten Aufnahmemodus entsprechen. Wie nachfolgend insbesondere an Hand der Figuren noch näher erläutert wird, können die Signale mehrerer Bildsensoren wieder miteinander kombiniert werden, um eine qualitativ hochwertige Aufnahme in einem bestimmten Aufnahmemodus zu erstellen.

Vorzugsweise sind die Bildteilungsmittel und die Filtermittel derart ausgebildet, dass sie das Objekt auf einen der Bildsensoren in einem Wellenlängenbereich abbilden, der im Wesentlichen dem Wellenlängenbereich des sichtbaren Lichts oberhalb einer vorgegebenen Grenzwellenlänge entspricht, wobei die Grenzwellenlänge vorzugsweise bei etwa 450nm liegt. Damit umfasst das von dem entsprechenden Sensor erfasste Signal zunächst einmal die für eine Fluoreszenzdiagnose erforderlichen Wellenlängen des sichtbaren Lichts, also insbesondere den relevanten orange-roten Bereich, wobei gleichzeitig das zur Fluoreszenzanregung verwendete Licht, welches eine zentrale Wellenlänge von beispielsweise 405nm aufweist, nicht mit erfasst wird und sich somit nicht störend auf die Fluoreszenzaufnahme auswirkt.

Gleichzeitig kann das von diesem Bildsensor erfasste Licht auch für eine Weißlichtaufnahme herangezogen werden, wobei sich allerdings das Fehlen der Wellenlängen aus dem blauen bzw. violetten Bereich unterhalb von 450nm negativ auf die Qualität der Weißlichtaufnahme auswirken würde.

Gemäß einer vorteilhaften Weiterbildung der Erfindung ist deshalb vorgesehen, dass die Bildteilungsmittel und die Filtermittel das Objekt auf einen weiteren der Bildsensoren in einem weiteren Wellenlängenbereich abbilden, der im Wesentlichen dem Wellenlängenbereich des sichtbaren Lichts unterhalb einer vorgegebenen Grenzwellenlänge entspricht, wobei die Grenzwellenlänge vorzugsweise bei etwa 450nm liegt und wobei der Wellenlängenbereich vorzugsweise eine Wellenlängenuntergrenze aufweist, die etwa im Bereich von 380nm liegt. Das Signal dieses Sensors, welches für die Fluoreszenzaufnahme ignoriert werden kann bzw. muss, kann dann mit dem Signal des zuvor erwähnten Sensors kombiniert werden, so dass insgesamt eine qualitativ hochwertige Weißlichtaufnahme erstellt werden kann, welche sämtliche Wellenlängen des sichtbaren Lichts umfasst.

Weiterhin sind die Bildteilungsmittel und die Filtermittel vorzugsweise derart ausgeführt, dass sie das Objekt auf einen dritten der Bildsensoren in einem Wellenlängenbereich abbilden, der im Wesentlichen dem Wellenlängenbereich des an das sichtbare Licht angrenzenden Infrarotbereichs entspricht. Das Signal dieses Sensors wird dann für die angesprochene Transilluminationsaufnahme verwendet.

Bei einer Ausgestaltung der Bildteilungsmittel und der Filtermittel, wie sie soeben erläutert wurde, wird also die Möglichkeit geschaffen, im Idealfall sogar Bilder in allen drei Aufnahmemodi zeitgleich mit Hilfe der zahnärztlichen Kamera zu erstellen. Grundsätzlich wäre es allerdings auch denkbar, das erfindungsgemäße Kamerahandstück derart ausgestalten, dass dieses mindestens drei Bildsensoren aufweist und die Bildteilungsmittel und die Filtermittel das Objekt auf den Bildsensoren in drei unterschiedlichen Farben, beispielsweise in den Farben rot, grün und blau abbilden. Wird das von den drei Sensoren erfasste Bild dann wiederum zusammengesetzt, so wird zwar nicht das zeitgleiche Erstellen von Aufnahmen in verschiedenen Aufnahmemodi ermöglicht, allerdings kann eine Weißlichtaufnahme in deutlich höherer Qualität realisiert werden, da die Nutzung von drei Bildsensoren zu einer entsprechenden Vervielfältigung der zur Verfügung stehenden Einzel-Pixel führt. Auch bei dieser Variante wird also gegenüber bislang bekannten zahnärztlichen Kamerahandstücken eine Verbesserung hinsichtlich der Bildaufnahme ermöglicht.

Wie bereits erwähnt, entspricht das von einem einzelnen Bildsensor erhaltene Signal noch nicht zwingend einem Bild in einem der drei zur Verfügung stehenden Aufnahmemodi. Stattdessen ist vorzugsweise vorgesehen, dass das Kamerahandstück eine Bilderstellungseinheit aufweist, welche auf Basis der von den digitalen Bildsensoren erfassten Daten Bilder entsprechend verschiedener Aufnahmemodi erstellt. Hierbei werden für zumindest einen Aufnahmemodus die Daten mehrerer Bildsensoren miteinander kombiniert.

Die Bildteilungsmittel können beispielsweise durch ein Prisma gebildet werden, wobei vorzugsweise an Flächen des Prismas Reflexionsschichten ausgebildet sind, welche jeweils Licht entsprechend einem der vorgegebenen Wellenlängenbereiche reflektieren. Diese speziellen Reflexionsschichten stellen also die eingangs erwähnten Filtermittel dar.

Alternativ zur Verwendung des Prismas wäre auch denkbar, in den Strahlengang des optischen Systems mehrere semi-transparente Filter einzubringen, welche jeweils Licht entsprechend einem jeweiligen Wellenlängen-Bereich reflektieren. Durch jeden der Filter wird also ein entsprechender Wellenlängenanteil des Lichts aus dem Strahlengang des optischen Systems ausgekoppelt und auf einen zugehörigen Sensor gerichtet.

Die Beleuchtungsmittel des erfindungsgemäßen zahnärztlichen Kamerahandstücks sind dabei ferner vorzugsweise dazu ausgebildet, das vor dem Eintrittsfenster des Kamerahandstücks befindliche Objekt entsprechend drei verschiedener Aufnahmemodi zu beleuchten, wobei die spektrale Zusammensetzung des von den Beleuchtungsmitteln abgegebenen Lichts für jeden Aufnahmemodus unterschiedlich ist.

Hierbei weisen die Beleuchtungsmittel jeweils unterschiedliche Lichtquellen auf, die jeweils einem Aufnahmemodus entsprechendes Licht abgeben, wobei vorzugsweise die zumindest einem Aufnahmemodus entsprechende Lichtquelle Bestandteil eines Aufsatzes ist, der lösbar am vorderen Ende des Kamerahandstücks angeordnet ist. In einem Synchronmodus, in dem parallel Aufnahmen entsprechend verschiedener Aufnahmemodi erstellt werden sollen, sind dann die Beleuchtungsmittel derart ausgebildet beziehungsweise werden derart angesteuert, dass das den verschiedenen Aufnahmemodi entsprechende Licht kombiniert abgegeben wird.

Hinsichtlich seines Aufbaus ist das Kamerahandstück vorzugsweise derart gestaltet, dass es eine längliche und rohrartig ausgestaltete Griffhülse aufweist, welche am vorderen Ende ein Lichteintrittsfenster aufweist, wobei das optische System innerhalb der Griffhülse angeordnet ist und das über das Lichteintrittsfenster eintretende Licht auf die Bildsensor-Anordnung richtet. Dabei ist vorzugsweise eine Flächennormale des Lichteintrittsfensters im Wesentlichen senkrecht zur Längsachse der Griffhülse ausgerichtet, wobei dann im vorderen Endbereich der Griffhülse ein Umlenkelement, beispielsweise ein Spiegel oder ein Prisma, angeordnet ist.

Nachfolgend soll die Erfindung an Hand der beiliegenden Zeichnungen näher erläutert werden. Es zeigen:
Figur 1 eine perspektivische Ansicht eines erfindungsgemäßen zahnärztlichen Kamerahandstücks;
Figur 2 einen Teilschnitt des vorderen Endbereichs des Kamerahandstücks ohne einen Aufsatz für die Transilluminationsaufnahme;
Figur 3 schematisch den grundsätzlichen Aufbau eines erfindungsgemäßen Kamerahandstücks;
Figur 4 eine erste erfindungsgemäße Variante zur Ausgestaltung des optischen Systems sowie der Sensor-Anordnung;
Figur 5 in vergrößerter Darstellung die Beeinflussung des von dem optischen System erfassten Lichts durch die erfindungsgemäßen Bildteilungsmittel;
Figur 6 eine Darstellung der von den verschiedenen Bildsensoren erfassten Wellenlängenbereiche;
Figur 7 eine Darstellung der Vorgehensweise zum Erstellen synchroner Aufnahmen in verschiedenen Aufnahmemodi und
Figur 8 eine zweite erfindungsgemäße Variante zur Ausgestaltung der Bildteilungsmittel sowie der Sensor-Anordnung.

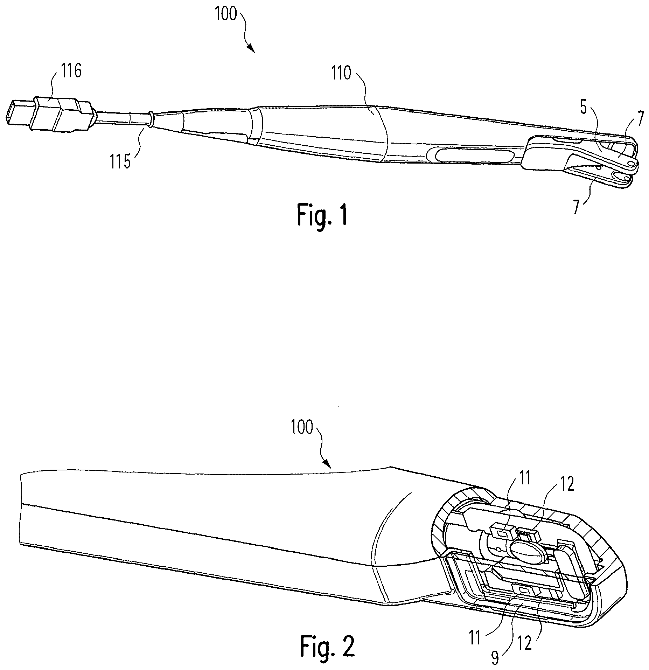

Figur 1 zeigt zunächst eine perspektivische Ansicht eines erfindungsgemäßen, allgemein mit dem Bezugszeichen 100 versehenen Kamerahandstücks. Dieses gleicht hinsichtlich seines Aufbaus grundsätzlich dem bspw. in der EP 2 452 614 A1 der Anmelderin beschriebenen Kamerahandstück. Es weist also zunächst eine längliche Griffhülse 110 auf, welche alle wesentlichen Komponenten zur Bildaufnahme beinhaltet und an ihrem rückseitigen Ende über ein Anschlusskabel 115 mit einem externen Gerät verbindbar ist. Wie in Figur 1 erkennbar, erfolgt vorzugsweise die Anbindung des Kamerahandstücks 100 mit Hilfe eines USB-Anschlusses 116 an einen PC. Bei diesem PC kann es sich insbesondere um einen PC eines zahnärztlichen Behandlungsplatzes oder einen sog. Hinterkopf-PC handeln. Mit Hilfe des Kamerahandstücks 100 erstellte Aufnahmen können dann unmittelbar auf einem Bildschirm des Behandlungsplatzes einem Patienten gezeigt werden. Dies erleichtert oftmals das Erläutern einer entsprechenden Diagnose sowie der sich hieraus ergebenden, durchzuführenden Behandlungsmaßnahmen. Alternativ zu einer Verbindung mit Hilfe eines USB-Anschlusses kann selbstverständlich auch eine kabellose Datenübertragung von dem Kamerahandstück 100 zu einem externen Gerät erfolgen. Beispielsweise kann dieses auch dauerhaft mit einer zahnärztlichen Behandlungseinheit verbunden sein.

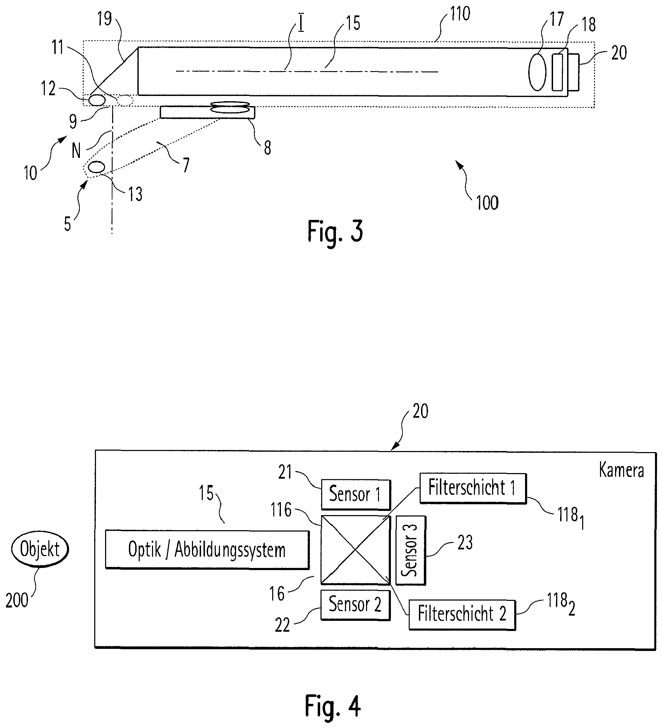

Die Griffhülse 110 des Kamerahandstücks 100 ist länglich ausgeführt, wobei die Bilderfassung am vorderen Ende der Griffhülse 110 erfolgt. Hierzu weist das Handstück 100 am vorderen Endbereich ein seitliches Lichteintrittsfenster 9 auf, welches auf das zu beobachtende Objekt, also bspw. den zu erfassenden Zahn oder Mundbereich des Patienten auszurichten ist. Die Flächennormale N (s. Figur 3) des Lichteintrittsfensters 9 ist somit im Wesentlichen senkrecht zur Längsachse I des Kamerahandstücks 100 ausgerichtet. Es hat sich gezeigt, dass bei einer derartigen Ausgestaltung die Handhabung des Kamerahandstücks 100 beim Erstellen intraoraler Aufnahmen erleichtert wird. Insbesondere ergibt sich eine ergonomisch deutlich günstigere Haltung, wenn die dem Mundraum zugewandten Zahnoberflächen oder die Kauflächen von Zähnen beobachtet werden sollen.

Das Lichteintrittsfenster 9 ist hierbei insbesondere im Teilschnitt von Figur 2 erkennbar, da hier ein am vorderen Ende des Kamerahandstücks 100 vorgesehener Aufsatz 5 nicht dargestellt ist bzw. das Kamerahandstück 100 in einem Zustand gezeigt ist, in dem dieser Aufsatz 5 entfernt wurde. Dieser Aufsatz 5 ist beim dargestellten Ausführungsbeispiel insbesondere für das Erstellen von Transilluminationsaufnahmen vorgesehen. Er weist - wie in Figur 1 erkennbar - zwei seitliche Arme 7 auf, an deren vorderen Enden jeweils später beschriebene Lichtquellen angeordnet sind. Beim Erstellen einer Transilluminationsaufnahme ist das Kamerahandstück 100 derart zu positionieren, dass das Sichtfenster 9 bspw. auf die Kaufläche eines zu beobachtenden Zahns ausgerichtet ist. Die Arme 7 mit den Lichtquellen sind dann jeweils zu beiden Seiten des zu untersuchenden Zahns angeordnet und deren Lichtquellen derart ausgerichtet, dass das für die Transillumination verwendete Licht seitlich in den Zahn eingekoppelt und durch diesen gestreut wird. Die Lichtquellen befinden sich hierbei jeweils anliegend an den unteren Seitenwänden das Zahn oder sogar im Bereich des Zahnfleisches, um das Licht möglichst homogen in den Zahn einzukoppeln. Diese beschriebene Anordnung der Komponenten zur Beleuchtung und zur Beobachtung des Zahns führt nicht nur zu einer sehr effizienten Einkopplung der für die Transillumination vorgesehenen Strahlung, sondern verhindert ferner auch, dass die Transilluminationsaufnahme durch externes Streulicht oder ungewünschte Reflexionen negativ beeinträchtigt wird. Entsprechende Aufsätze sind detailliert in der EP 2 452 614 A1 beschrieben.

Eine derartige seitliche Lichteinkopplung ist also in erster Linie für die erwähnten Transilluminationsaufnahmen von Vorteil. Beim Erstellen einer Weißlichtaufnahme oder einer Aufnahme im Rahmen einer Fluoreszenzdiagnose hingegen ist keine seitliche Lichteinkopplung vorgesehen, sondern das Licht wird vorzugsweise aus Richtung des Sichtfensters 9 auf den Zahn bzw. allgemein auf das zu untersuchende Objekt gerichtet. Damit wird also bei der Transillumination eine im wesentlichen senkrechte Beleuchtungsrichtung im Vergleich zur Beleuchtungsrichtung für die beiden anderen Aufnahmemodi realisiert.

Die Tatsache, dass der Aufsatz 5 nur für einen der drei Aufnahmemodi benötigt wird, bedeutet, dass unter gewissen Umständen auch auf diesen verzichtet werden kann, was dann ggf. wiederum eine etwas flexiblere Positionierung der Kamera 100 für die weiteren Aufnahmearten ermöglicht. Vorzugsweise ist deshalb der Aufsatz 5 derart ausgestaltet, dass er lösbar auf das vordere Ende des Kamerahandstücks 100 aufgesetzt werden und bspw. mit diesem verrastet werden kann. Dabei kann ferner auch vorgesehen sein, dass unterschiedlich gestaltete Aufsätze 5 zur Verfügung stehen, die abhängig davon zum Einsatz kommen, welcher Typ eines Zahns mit Hilfe des Kamerahandstücks 100 erfasst werden soll. Auch in diesem Zusammenhang wird auf die EP 2 452 614 A1 verwiesen, in der mehrere unterschiedlich gestaltete entsprechende Aufsätze beschrieben sind. Diese können in vergleichbarer Weise auch bei dem vorliegenden erfindungsgemäßen Kamerahandstück 100 zum Einsatz kommen. Ferner kann auch vorgesehen sein, dass verschiedene Aufsätze 5 zur Verfügung stehen, die sich hinsichtlich der Länge bzw. Größe der Arme 7 unterscheiden, so dass abhängig von dem Alter des Patienten und damit der Größe der zu untersuchenden Zähne jeweils optimal geeignete Aufsätze 7 ausgewählt werden können.

Nachdem bislang in erster Linie der mechanische Aufbau des Kamerahandstücks 100 beschrieben wurde, sollen nachfolgend die für die Bildaufnahme erforderlichen Komponenten anhand von Figur 3 erläutert werden. Gezeigt ist hierbei eine schematische Darstellung der Ausgestaltung des Handstücks 100, wobei zunächst stark vereinfacht diejenigen Bestandteile gezeigt sind, die für das Erstellen einer Aufnahme in den verschiedenen Aufnahmemodi verantwortlich sind.

Hierbei ist zunächst wie oben beschrieben erforderlich, dass abhängig von dem gewählten Aufnahmemodus das zu untersuchende Objekt in geeigneter Weise beleuchtet wird. Das Kamerahandstück 100 weist dementsprechend drei verschieden gestaltete Lichtquellen bzw. Leuchtmittel auf, die je nach Aufnahmemodus aktiviert sind.

Es handelt sich hierbei zunächst um eine erste Lichtquelle 11 zur Nutzung während eines Weißlicht-Aufnahmemodus. Diese Lichtquelle 11 soll also primär das zu untersuchende Objekt mit Licht aus dem sichtbaren Wellenlängenbereich beleuchten, wobei die spektrale Zusammensetzung des Lichts derart ist, dass sich insgesamt weißes Mischlicht ergibt. Denkbar wäre also insbesondere die Verwendung einer Kombination verschiedenfarbiger LEDs oder einer blauen LED, deren Licht durch geeignete Phosphore in Weißlicht konvertiert wird. Wichtig ist, dass es sich bei den Lichtquellen für die drei Aufnahmemodi nicht zwingend jeweils um Einzellichtquellen handeln muss, sondern dass diese durchaus wiederum durch eine Kombination mehrere Lichtquellen gebildet sein können.

Für die Fluoreszenzdiagnose wird im Gegensatz zur Weißlichtaufnahme die zweite Lichtquelle 12 benutzt, die den zu untersuchenden Zahn mit einer nicht sichtbaren, kurzwelligen Anregungsstrahlung im Bereich von etwa 405nm beleuchtet. Licht einer derartigen Wellenlänge veranlasst kariöses Zahngewebe, eine Fluoreszenzstrahlung im Bereich oberhalb von 480nm, also im orange-roten sichtbaren Bereich abzugeben. Dabei werden in der Literatur unterschiedliche Wellenlängen für die Anregungsstrahlung vorgeschlagen, wobei diese jedoch im Allgemeinen immer im blauen bzw. violetten Bereich liegen.

Beide Lichtquellen 11 und 12 sind vorzugsweise derart positioniert, dass sie das zu beobachtende Objekt aus der im Wesentlichen gleichen Richtung beleuchten, aus der dieses durch das optische System 15 der Kamera 100 erfasst wird. D.h., die Positionierung dieser Lichtquellen 11 und 12 wird im Bereich des Lichteintrittsfensters 9, bspw. verteilt an dessen Umfang erfolgen.

Die dritten Lichtquellen 13 schließlich sind diejenigen, die an den Enden der Arme 7 des Aufsatzes 5 angeordnet sind und seitlich Licht in den Zahn im Rahmen einer Transilluminationsaufnahme einkoppeln sollen. Wie erläutert sollen diese Lichtquellen 13 in erster Linie Licht im Infrarotbereich oberhalb von 780nm abgeben, insbesondere Licht mit einer Wellenlänge von etwa 850nm, da hier die innerhalb des Zahns resultierende Lichtstreuung besonders gut das Erkennen von Karies ermöglicht. Den Lichtquellen 13 können geeignete Lichtabgabe- bzw. Lichteinkoppelelemente zugeordnet sein, mit deren Hilfe die Lichteinstrahlung in den Zahn optimiert wird. Es kann sich hierbei um aus Silikon bestehende Elemente mit speziellen Strukturierungen handeln, die dann an der Außenfläche des zu untersuchenden Zahns anliegen.

Insgesamt weisen also die Beleuchtungsmittel 10 des Kamerahandstücks 100 drei verschiedene Lichtquellen 11, 12 und 13 auf, die je nach Beleuchtungsmodus bzw. Aufnahmemodus das zu beobachtende Objekt in unterschiedlicher Weise beleuchten. Hierbei ist anzumerken, dass insbesondere für die ersten beiden Lichtquellen 11 und 12 nicht zwingend eine physische Trennung der Leuchtmittel vorliegen muss. Da die spektrale Zusammensetzung des Lichts für die jeweils verschiedenen Aufnahmemodi allerdings nicht überlappt, werden letztendlich jeweils separate Leuchtmittel zum Einsatz kommen, die dann allerdings ggf. auf einer gemeinsamen Platine oder einem anderen gemeinsamen Träger angeordnet sind.

In Figur 3 ist weiterhin schematisch dargestellt, dass für den abnehmbar ausgestalteten Aufsatz 5 Stromversorgungsmittel 8 vorgesehen sein können, um die Leuchtmittel 13 des Aufsatzes 5 für die Transilluminationsaufnahme mit Energie zu versorgen. Bevorzugt kommen hierbei Stromversorgungsmittel zum Einsatz, die eine induktive, also eine kabellose Stromversorgung ermöglichen, was zu Vorteilen hinsichtlich der Möglichkeiten, das Kamerahandstück 100 zu reinigen, führt. Ferner können - in den Figuren nicht näher dargestellte - Sensoren vorgesehen sein, mit deren Hilfe erkannt wird, ob ein Aufsatz 5 aufgesteckt wurde oder nicht. Hiermit lässt sich zusätzlich auch eine Zuordnung oder eindeutige Identifizierung des Aufsatzes 5 ermöglichen.

Neben den Lichtquellen 11 bis 13 sind in Figur 3 auch die für die Bilderfassung verantwortlichen Komponenten erkennbar. Diese beinhalten zunächst eine digitale Bildsensor-Anordnung 20, die innerhalb der Griffhülse 110 des Handstücks 100 angeordnet ist und mit einer nicht näher gezeigten Steuerelektronik verbunden ist. Dieser Bildsensor-Anordnung 20 ist ein optisches System 15 vorgeschaltet, mit dessen Hilfe das über das Lichteintrittsfenster 9 einfallende Licht auf die Bildsensor-Anordnung 20 gerichtet wird. Dieses optische System 15 ist im vorliegenden Fall stark vereinfacht dargestellt, wobei es entsprechend der Darstellung eine der Bildsensor-Anordnung 20 vorgeschaltete Fokussierlinse 17, ein am vorderen Ende des Handstücks 100 zur Lichtumlenkung vorgesehenes Umlenkprisma 19 sowie Filtermittel 18 aufweist. Es handelt sich wie bereits erwähnt um eine stark vereinfachte Darstellung des optischen Systems 15, welches darüber hinaus gehend auch mehrere weitere Linsen und Blenden aufweisen wird, um eine kontrastreiche, scharfe Abbildung des Objekts auf der Bildsensor-Anordnung 20 zu gewährleisten. Auf die Darstellung dieser weiteren Komponenten wurde allerdings verzichtet, da diese nicht Hauptbestandteil der Erfindung sind. Anzumerken ist allerdings, dass alternativ zu dem Umlenkprisma 19 selbstverständlich auch ein Umlenkspiegel zum Einsatz kommen könnte.

Die Erfindung betrifft insbesondere die Ausgestaltung der Filtermittel 18 sowie der Bildsensor-Anordnung 20 und kann insbesondere der Darstellung von Figur 4 entnommen werden. Die wesentliche Besonderheit besteht hierbei darin, dass im Gegensatz zu bislang bekannten Lösungen nicht ein einzelner Bildsensor zum Einsatz kommt, sondern stattdessen eine Anordnung bestehend aus drei digitalen Bildsensoren 21 bis 23 vorgesehen ist. Das von dem zu beobachtenden Objekt 200 abgegebene Licht wird hierbei durch das optische System 15 der Kamera 100 auf jeden der drei Bildsensoren 21 bis 23 abgebildet, allerdings derart, dass jeder Bildsensor 21 bis 23 lediglich Licht innerhalb eines bestimmten Wellenlängenbereichs empfängt.

Entsprechend der Darstellung von Figur 4 ist hierzu vorgesehen, dass das optische System 15 Bildteilungsmittel 16 in Form eines Prismas 116 aufweist. Dieses ist intern mit Trennschichten 118₁ und 118₂ ausgebildet, die jeweils einen Teil des Lichts aus dem optischen Pfad auskoppeln und derart umlenken, dass dieser Anteil des Lichts auf einen der Sensoren 21 bis 23 geworfen wird. Diese Trennschichten 118₁, 118₂ sind dabei derart ausgestaltet, dass sie jeweils das Licht eines bestimmten zusammenhängenden Wellenlängenbereichs auskoppeln und auf den zugehörigen Sensor 21 bis 23 werfen. Damit bilden sie zusätzlich auch Filtermittel 18, die das Objekt 200 auf jeden Sensor 21 bis 23 jeweils in einem vorgegebenen Wellenlängenbereich abbilden.

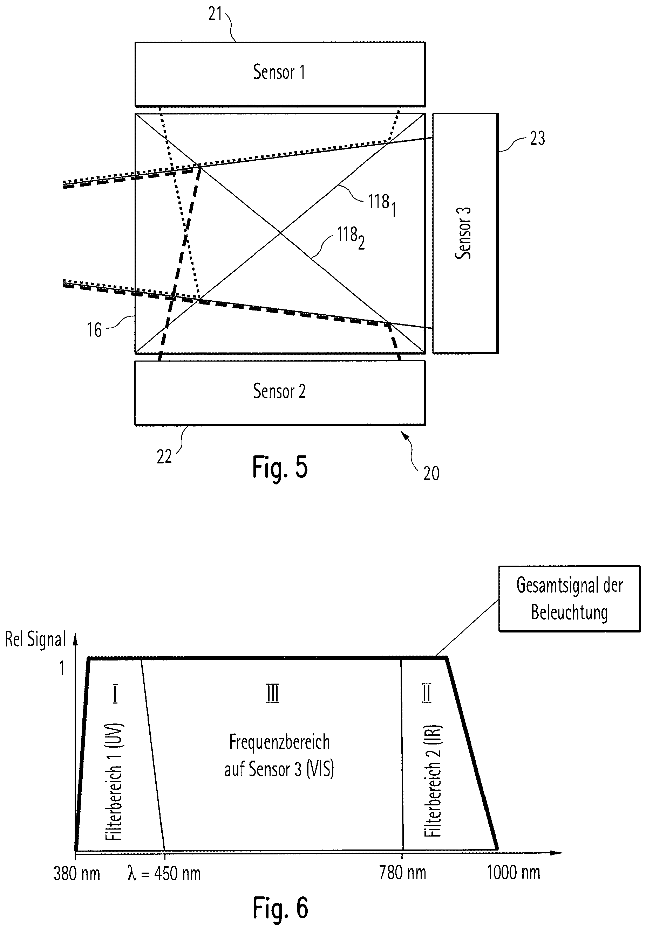

Die Auskopplung des Lichts ist vergrößert in Figur 5 dargestellt, wobei erkennbar ist, dass die erste Reflexionsschicht 118₁, welche in der Darstellung diagonal von links unten nach rechts oben verläuft, das einfallende Lichtbündel innerhalb eines entsprechend zugehörigen Wellenlängenbereichs derart reflektiert, dass dieses auf den ersten Sensor 21 umgelenkt wird. Die zweite Reflexionsschicht 118₂ wiederum verläuft diagonal von links oben nach rechts unten und reflektiert das Licht eines weiteren Wellenlängenbereichs derart, dass das zugehörige Lichtbündel auf den zweiten Sensor 22 geworfen wird. Die restlichen Wellenlängenanteile können beide Reflexionsschichten 118₁ und 118₂ im Wesentlichen ungehindert passieren und gelangen als drittes Lichtbündel auf den dritten Sensor 23.

Selbstverständlich wäre es auch denkbar, das durch das Sichtfenster 9 einfallende Lichtbündel - ohne vorherigen Filter - auf jeden der drei Sensoren 21 bis 23 zu lenken und unmittelbar vor jedem Bildsensor 21-23 jeweils einen geeigneten Filter vorzusehen. Die dargestellte Variante ist allerdings zu bevorzugen, da hier tatsächlich durch die Reflexionsschichten 118₁ und 118₂ immer nur derjenige Wellenlängenanteil des Lichts ausgekoppelt wird, der für den entsprechenden Sensor 21-23 auch tatsächlich relevant ist. Damit wird in diesem Fall das zu Verfügung stehende Licht deutlich effizienter genutzt.

Figur 6 zeigt, welche Wellenlängenbereiche des Lichts jeweils durch die verschiedenen Sensoren erfasst werden.

Das von dem ersten Sensor 21 erfasste Licht liegt hierbei im kurzwelligen Bereich des sichtbaren Lichts, unterhalb einer Grenzwellenlänge λ von etwa 450nm. Idealerweise sollte dieser Wellenlängenbereich I eine Untergrenze aufweisen, die mit dem unteren Ende des sichtbaren Lichts übereinstimmt. Im vorliegenden Fall ist dementsprechend eine Untergrenze von etwa 380nm vorgesehen.

Der zweite Sensor 22 wiederum ist dazu vorgesehen, den Wellenlängenbereich II zu erfassen, der den Infrarotbereich des Lichts bis zu etwa 1000nm umfasst. Dieser Wellenlängenbereich ist insbesondere für die Transilluminationsaufnahme von Interesse. Er ist an seiner Unterseite durch das sichtbare Licht, also bei etwa 780nm begrenzt.

Der verbleibende Wellenlängenbereich III entspricht dann dem sichtbaren Licht - mit Ausnahme des durch den ersten Sensor 21 erfassten Wellenlängenbereichs I - und wird durch den dritten Sensor 23 erfasst.

In Kombination werden also die drei digitalen Bildsensoren 21 bis 23 Licht über einen Wellenlängenbereich von etwa 380nm bis 1000nm erfassen. Soll nun eine Aufnahme eines zu untersuchenden Zahns in einem der drei Aufnahmemodi erstellt werden, so muss das Signal des entsprechenden Sensors 21 bis 23 beziehungsweise der entsprechenden Sensoren 21 bis 23 herangezogen werden. Es ist nämlich nicht grundsätzlich das Signal eines einzelnen Bildsensors für einen einzelnen Aufnahmemodus relevant, sondern es kann auch eine Kombination der Signale mehrerer Sensoren 21 bis 23 herangezogen werden. Dabei ergibt sich der in der nachfolgenden Tabelle gezeigte Zusammenhang.

| Aufnahmemodus | relevante Wellenlängen (Wellenlängenbereich) | verwendete Sensoren |
|---|---|---|
| Weißlichtaufnahme | gesamtes sichtbares Licht (I + III) | Sensor 1 + Sensor 3 |
| Fluoreszenzaufnahme | sichtbares Lichts ohne kurzwelligen Anteil (III) | Sensor 3 |
| Transillumination | Infrarotlicht (II) | Sensor 2 |

Für eine Weißlichtaufnahme wird also nicht nur das Signal des dritten Sensors 23 berücksichtigt, sondern zusätzlich auch das Signal des ersten Sensors 21. In Kombination umfassen nämlich beide Sensoren den gesamten Wellenlängenbereich des sichtbaren Lichts, sodass eine qualitativ hochwertige Aufnahme im Weißlichtmodus erstellt werden kann.

Soll hingegen eine Aufnahme im Fluoreszenzdiagnose-Modus erstellt werden, so wird nur das Signal des dritten Sensors 23 berücksichtigt. Wie erläutert erfasst dieser Sensor 23 den Wellenlängenbereich III, der den orange-roten Anteil des sichtbaren Lichts umfasst, also denjenigen Anteil, in dem kariöses Gewebe in Reaktion auf eine Anregungsstrahlung eine Fluoreszenzstrahlung emittiert. Die kurzwelligen Wellenlängen des Bereichs I, der somit die für die Fluoreszenzanregung verwendete Wellenlänge von etwa 405nm einschließt, sind für eine derartige Fluoreszenzdiagnose hingegen eher uninteressant beziehungsweise wirken sich sogar störend aus, sodass das Signal des Sensors 23 ausreichend ist und eine qualitativ hochwertige Aufnahme in diesem Modus ermöglicht.

Für die Transilluminationsaufnahme wiederum wird wie eingangs erwähnt bevorzugt das im Zahn gestreute Licht im Infrarotbereich genutzt. Es handelt sich hierbei um das Signal, das durch den Sensor 22 erfasst wird, sodass sichergestellt ist, dass durch die Transillumination tatsächlich kein sichtbares Licht, sondern lediglich das gestreute Infrarotlicht berücksichtigt wird.

Da aufgrund der erfindungsgemäßen Ausgestaltung des optischen Systems 15 und der Bildsensor-Anordnung 20 alle drei Sensoren 21 bis 23 parallel bzw. zeitgleich Signale zur Verfügung stellen, kann durch das Auslesen sowie durch gegebenenfalls geeignetes Kombinieren der Sensorsignale zeitgleich eine Aufnahme in jedem der drei denkbaren Aufnahmemodi erstellt werden. Dies gilt nicht nur für Einzelbildaufnahmen, sondern auch für kontinuierliche Aufnahmen, also für das Erstellen von Videos. Die letztendlich hiermit erhaltenen parallelen Aufnahmen bzw. Videos in den verschiedenen Aufnahmemodi können dann nebeneinander auf einem Bildschirm dargestellt werden oder gegebenenfalls wiederum miteinander zu einem Einzelbild oder einem einzigen Video kombiniert werden, wobei hier nun allerdings dann die Möglichkeit besteht, die für die Fluoreszenzdiagnose oder für die Transillumination relevanten Informationen in spezieller Weise - beispielsweise durch eine entsprechend Farbgebung oder dergleichen - in dem Bild hervorzuheben, sodass diese Informationen entsprechend gut erkennbar sind.

Letztendlich wird also eine äußerst komfortable Möglichkeit geschaffen, mehrere Diagnoseverfahren parallel in einem optischen Untersuchungsverfahren zu kombinieren, um eine aussagekräftige optische Kariesdiagnose zu ermöglichen.

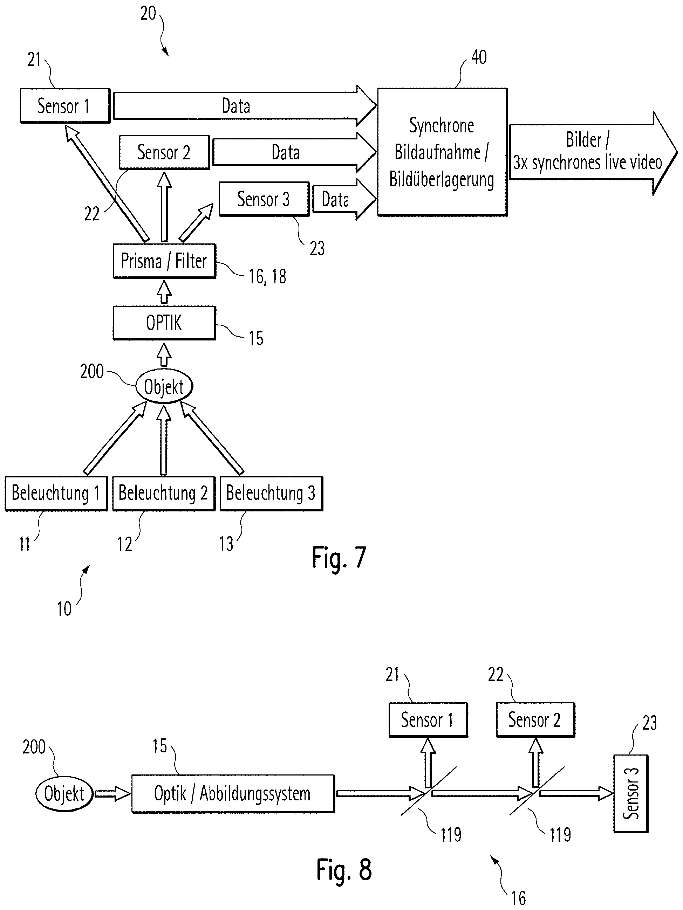

Voraussetzung für das Erstellen einer Aufnahme in einem der drei Aufnahmemodi ist selbstverständlich, dass - wie oben bereits beschrieben - das zu untersuchende Objekt beziehungsweise der Zahn mit entsprechend geeignetem Licht beleuchtet ist. Schematisch ist dies nochmal in Figur 7 dargestellt, wobei hier die Beleuchtungsmittel 10 durch drei separate Lichtquellen 11 bis 13 realisiert sind, die dann gemeinsam das zu beobachtende Objekt 200 beleuchten. Mit Hilfe der erfindungsgemäßen Bildteilungsmittel 16 und Filtermittel 18 wird dann das Licht auf die drei Bildsensoren 21 bis 23 verteilt. Die von den Sensoren 21 bis 23 gelieferten Daten werden durch eine entsprechende Bilderstellungseinheit 40 ausgewertet und gegebenenfalls wiederum kombiniert, um synchrone Aufnahmen zu erhalten. Diese Bilderstellungseinheit 40 kann dabei unmittelbar in das zahnärztliche Kamerahandstück 100 integriert sein. Denkbar wäre allerdings auch, dass die von den drei Sensoren 21 bis 23 gelieferten Daten parallel als Rohdaten an ein externes Gerät durch die Kamera 100 übermittelt werden und erst dort weiterverarbeitet werden. Diese externe Einheit kann dann beispielsweise durch den bereits erwähnten PC des zahnärztlichen Behandlungsplatzes realisiert sein.

Die Unterteilung der Beleuchtungsmittel 10 in drei separate Lichtquellen 11 bis 13, die jeweils individuell aktivierbar sind, bringt dabei den Vorteil mit sich, dass nicht zwingend grundsätzlich parallele Aufnahmen in allen drei Aufnahmemodi erstellt werden müssen. Beispielsweise kann in gewissen Situationen gewünscht sein, dass lediglich zeitlich eine Weißlichtaufnahme sowie eine Aufnahme im Rahmen einer Fluoreszenzdiagnose erstellt werden. In diesem Fall ist es ausreichend, die ersten beiden Lichtquellen 11 und 12 zu aktivieren, die für eine Transilluminationsaufnahme benötigte Infrarotlichtquelle 13 hingegen auszuschalten bzw. den Aufsatz 5 von dem Kamerahandstück 100 zu entfernen. Soll wiederum lediglich eine Weißlichtaufnahme erstellt werden, so ist es ausreichend, ausschließlich die erste Lichtquelle 11 zu aktivieren. Trotz allem wird in diesem Fall das vom ersten Sensor 21 und von dem dritten Sensor 23 erfasste Signal miteinander kombiniert werden, um für alle Wellenlängen des sichtbaren Lichts entsprechende Informationen zu erhalten.

Die Unterteilung der Leuchtmittel 10 in einzeln ansteuerbare individuelle Lichtquellen 11 bis 13 ist auch insofern sinnvoll, als die für die Transilluminationsaufnahmen benötigten Lichtquellen 13 vorzugsweise an dem auswechselbaren Aufsatz 5 und damit abnehmbar von dem Kamerahandstück 100 positioniert sind. Grundsätzlich ist allerdings nicht zwingend erforderlich, einzeln ansteuerbare Lichtquellen zu verwenden. Alternativ hierzu könnte also auch eine einzige Lichtquelle verwendet werden, die sämtliche für die drei Aufnahmen benötigten Wellenlängen abdeckt. Eine derartige Lichtquelle müsste also zunächst Weißlicht im sichtbaren Bereich abgeben, zusätzlich jedoch auch Licht im Infrarotbereich für die Transilluminationsaufnahme sowie Licht im UV-Bereich für die Fluoreszenzdiagnose.

Figur 8 zeigt eine zweite Variante der erfindungsgemäßen Ausgestaltung des optischen Systems 15 und der Bildsensor-Anordnung 20. Der wesentliche Unterschied im Vergleich zur ersten Variante gemäß Figur 4 besteht hierbei darin, dass anstelle des Prismas 116 mit den Filterschichten 118₁ und 118₂ zur Bildteilung semitransparente Spiegel 119 zum Einsatz kommen, die im Strahlengang des optischen Systems 15 hintereinander angeordnet sind. Jeder der Spiegel 119 reflektiert hierbei den zugehörigen Wellenlängenbereich des Lichts derart, dass dieser Anteil seitlich aus dem Strahlengang ausgekoppelt wird und auf den zugehörigen Sensor 21 bis 23 trifft. Die weitere Funktionsweise ist dann identisch zu derjenigen, welche an Hand der Figuren 4 bis 6 erläutert wurde. Wiederum erfasst also jeder der drei Sensoren 21 bis 23 Licht in einem bestimmten Wellenlängenbereich, wobei diese Bereiche derart gewählt sind, dass sie entweder dem für einen Aufnahmemodus relevanten Wellenlängenbereich des Lichts entsprechen oder derart miteinander kombiniert werden können, dass eine Aufnahme in einem der drei Aufnahmemodi erhalten werden kann.

Anzumerken ist in diesem Zusammenhang, dass bislang die Erfindung immer in Bezug auf drei parallel zur Verfügung stehende Aufnahmemodi erläutert wurde. Allerdings kann die Erfindung bereits in vorteilhafter Weise genutzt werden, wenn eine zahnärztliche Kamera lediglich zwei Aufnahmemodi zur Verfügung stellt. Soll beispielsweise grundsätzlich auf die Möglichkeit der Transillumination verzichtet werden, so wäre nach wie vor die Nutzung von zwei getrennten Bildsensoren, die den Sensoren 21 und 23 entsprechen, sinnvoll. Wird das Licht wie erläutert durch geeignete Bildteilungsmittel und Filtermittel in zwei Strahlenbündel aufgeteilt, die den oben beschriebenen Bereichen I und III entsprechen, so bestehet wiederum die Möglichkeit, zeitgleich bzw. parallel eine Weißlichtaufnahme sowie eine Aufnahme im Rahmen einer Fluoreszenzdiagnose zu erstellen. Analoges gilt für jede beliebige Kombination von zwei Aufnahmemodi.

Die bislang beschriebene Aufteilung des erfassten Lichts in die drei Wellenlängenbereiche I bis III ist also insofern von Vorteil, als in komfortabler Weise zeitgleich Bilder beziehungsweise Liveaufnahmen in jedem der drei Aufnahmemodi erhalten werden können. Die erfindungsgemäße Aufteilung des Lichts und Erfassung durch drei separate Sensoren könnte allerdings auch dazu genutzt werden, jeweils den drei Primärfarben entsprechende Lichtanteile auf die Sensoren 21 bis 23 zu werden. Es ist in diesem Fall dann zwar keine einem jeweiligen Aufnahmemodus entsprechende Signalkombination möglich, allerdings können die Daten der drei Sensoren 21 bis 23 dann wiederum zu einer Weißlichtaufnahme kombiniert werden. Die Qualität dieser resultierenden Weißlichtaufnahme ist deutlich erhöht, da durch die Nutzung mehrerer Bildsensoren auch die Anzahl der zur Verfügung stehenden Pixel entsprechend vervielfacht wird. Damit kann bei dieser Variante dann eine Aufnahme mit einer deutlich verbesserten Bildauflösung erstellt werden.

Insgesamt eröffnet somit die vorliegende Erfindung die Möglichkeit, eine zahnärztliche Kamera flexibler zu nutzen und entweder parallel Bilder oder Videos in verschiedenen Aufnahmemodi zu erstellen oder die Qualität einer Weißlichtaufnahme deutlich zu erhöhen.

## Patentansprüche

1. Zahnärztliches Kamerahandstück (100) zum Erstellen intraoraler Aufnahmen, aufweisend:
• Beleuchtungsmittel (10) zum Beleuchten eines vor einem Eintrittsfenster (9) des Kamerahandstücks (100) befindlichen Objekts (200),
• ein optisches System (15) zum Abbilden des Objekts (200) auf eine Bildsensor-Anordnung (20),
• eine Bildsensor-Anordnung (20), welche zumindest zwei getrennte digitale Bildsensoren (21, 22, 23) zum Erfassen des mit Hilfe des optischen Systems (15) abgebildeten Objekts (200) aufweist,
wobei das optische System (15) Bildteilungsmittel (16) aufweist, welche das Objekt (200) auf jeden der Bildsensoren (21, 22, 23) abbilden, sowie Filtermittel (18), welche derart ausgebildet sind, dass das Objekt (200) auf jedem Bildsensor (21, 22, 23) jeweils in einem vorgegebenen Wellenlängenbereich (I, II, III) abgebildet wird, wobei sich die Wellenlängenbereiche für die Bildsensoren (21, 22, 23) unterscheiden.

2. Zahnärztliches Kamerahandstück nach Anspruch 1,
**dadurch gekennzeichnet,**
**dass** die Bildteilungsmittel (16) und die Filtermittel (18) das Objekt (200) auf einen der Bildsensoren (23) in einem Wellenlängenbereich (III) abbilden, der im Wesentlichen dem Wellenlängenbereich des sichtbaren Lichts oberhalb einer vorgegebenen Grenzwellenlänge (λ) entspricht, wobei die Grenzwellenlänge (λ) vorzugsweise bei etwa 450nm liegt.

3. Zahnärztliches Kamerahandstück nach Anspruch 1 oder 2,
**dadurch gekennzeichnet,**
**dass** die Bildteilungsmittel (16) und die Filtermittel (18) das Objekt (200) auf einen der Bildsensoren (21) in einem Wellenlängenbereich (I) abbilden, der im Wesentlichen dem Wellenlängenbereich des sichtbaren Lichts unterhalb einer vorgegebenen Grenzwellenlänge (λ) entspricht, wobei die Grenzwellenlänge (λ) vorzugsweise bei etwa 450nm liegt,
und wobei der Wellenlängenbereich (I) vorzugsweise eine Wellenlängenuntergrenze aufweist, welche im Bereich von etwa 380nm liegt.

4. Zahnärztliches Kamerahandstück nach einem der Ansprüche 1 bis 3,
**dadurch gekennzeichnet,**
**dass** die Bildteilungsmittel (16) und die Filtermittel (18) das Objekt (200) auf einen der Bildsensoren (22) in einem Wellenlängenbereich (II) abbilden, der im Wesentlichen dem Wellenlängenbereich des an das sichtbare Licht angrenzenden Infrarotbereichs entspricht.

5. Zahnärztliches Kamerahandstück nach Anspruch 1,
**dadurch gekennzeichnet,**
**dass** dieses mindestens drei Bildsensoren (21, 22, 23) aufweist und die Bildteilungsmittel (16) und die Filtermittel (18) das Objekt (200) auf den Bildsensoren (21,22, 23) in drei unterschiedlichen Farben, vorzugsweise in den Farben Rot, Grün und Blau abbilden.

6. Zahnärztliches Kamerahandstück nach einem der vorherigen Ansprüche,
**dadurch gekennzeichnet,**
**dass** dieses eine Bilderstellungseinheit (40) aufweist, welche auf Basis der von den digitalen Bildsensoren (21, 22, 23) erfassten Daten Bilder entsprechend verschiedener Aufnahmemodi erstellt.

7. Zahnärztliches Kamerahandstück nach Anspruch 6,
**dadurch gekennzeichnet,**
**dass** für zumindest einen Aufnahmemodus die Daten mehrerer Bildsensoren (21, 22, 23) kombiniert werden.

8. Zahnärztliches Kamerahandstück nach einem der vorherigen Ansprüche,
**dadurch gekennzeichnet,**
**dass** die Bildteilungsmittel (16) durch mindestens ein Prisma (116) gebildet werden.

9. Zahnärztliches Kamerahandstück nach Anspruch 8,
**dadurch gekennzeichnet,**
**dass** an Flächen des Prismas (116) Reflexionsschichten (118₁, 118₂) ausgebildet sind, welche jeweils Licht entsprechend einem der vorgegebenen Wellenlängenbereiche (I, II, III) reflektieren.

10. Zahnärztliches Kamerahandstück nach einem der Ansprüche 1 bis 7,
**dadurch gekennzeichnet,**
**dass** die Bildteilungsmittel (16) durch in dem Strahlengang des optischen Systems (15) angeordnete semi-transparente Filter (119) gebildet sind, welche jeweils Licht entsprechend einem der vorgegebenen Wellenlängenbereiche (I, II, III) reflektieren.

11. Zahnärztliches Kamerahandstück nach einem der vorherigen Ansprüche,
**dadurch gekennzeichnet,**
**dass** die Beleuchtungsmittel (10) dazu ausgebildet sind, das vor dem Eintrittsfenster (9) des Kamerahandstücks (100) befindliche Objekt (200) entsprechend drei verschiedener Aufnahmemodi zu beleuchten, wobei die spektrale Zusammensetzung des von den Beleuchtungsmitteln (10) abgegebenen Lichts für jeden Aufnahmemodus unterschiedlich ist.

12. Zahnärztliches Kamerahandstück nach Anspruch 11,
**dadurch gekennzeichnet,**
**dass** die Beleuchtungsmittel (10) unterschiedliche Lichtquellen (11, 12, 13) aufweisen, die jeweils einem Aufnahmemodus entsprechendes Licht abgeben, wobei vorzugsweise die einem bestimmten Aufnahmemodus entsprechende Lichtquelle (13) Bestandteil eines Aufsatzes (5) ist, der lösbar am vorderen Ende des Kamerahandstücks (100) angeordnet ist.

13. Zahnärztliches Kamerahandstück nach Anspruch 11 oder 12,
**dadurch gekennzeichnet,**
**dass** die Beleuchtungsmittel (10) dazu ausgebildet sind, in einem Synchronmodus das zumindest zwei verschiedenen Aufnahmemodi entsprechende Licht kombiniert abzugeben.

14. Zahnärztliches Kamerahandstück nach einem der vorherigen Ansprüche,
**dadurch gekennzeichnet,**
**dass** dieses eine längliche und rohrartig ausgestaltete Griffhülse aufweist, welche am vorderen Ende ein Lichteintrittsfenster aufweist,
wobei das optische System (15) zum Abbilden des Objekts auf den Bildsensor (20) sowie der Bildsensor (20) innerhalb der Griffhülse angeordnet sind und das optische System (15) das über das Lichteintrittsfenster eintretende Licht auf den Bildsensor (20) lenkt.

15. Zahnärztliches Kamerahandstück nach Anspruch 14,
**dadurch gekennzeichnet,**
**dass** eine Flächennormale des Lichteintrittsfensters im Wesentlichen senkrecht zur Längsachse der Griffhülse ausgerichtet ist und im vorderen Ende der Griffhülse ein Umlenkelement, beispielsweise ein Spiegel oder ein Prisma angeordnet ist.
